Europäisches Patentamt

European Patent Office

Office européen des brevets

(19)

(11) Numéro de publication: **0 169 601**

**A1**

## (12) DEMANDE DE BREVET EUROPEEN

(21) Numéro de dépôt: **85201099.0**

(22) Date de dépôt: **08.07.85**

(51) Int. Cl.⁴: **A 61 K 37/34**
**A 61 K 37/02**
**//(A61K37/34, 31:71),**
**(A61K37/02, 31:71)**

(30) Priorité: **19.07.84 BE 213354**

(43) Date de publication de la demande:
**29.01.86 Bulletin 86/5**

(84) Etats contractants désignés:
**BE CH DE FR GB IT LI LU NL SE**

(71) Demandeur: **S.S.M. INTERNATIONAL CHEMICAL COMPANY LIMITED**
**Lot 18**
**Kingstown(VC)**

(71) Demandeur: **SPEECKE, André**
**Rue Bahoux, 81 Route de Bastogne**
**B-6680 Manhay(BE)**

(72) Inventeur: **Speecke, André**
**Rue Bahous, 81 Route de Bastogne**
**B-6680 Manhay(BE)**

(74) Mandataire: **De Brabanter, Maurice et al,**
**Bureau VANDER HAEGHEN 63 Avenue de la Toison d'Or**
**B-1060 Bruxelles(BE)**

(54) Composition ocytocique.

(57) L'invention est relative à une composition ocytocique pour usage vétérinaire, qui permet de traiter les infections de la matrice et les péritonites, après une mise bas des jeunes, sans réduire la lactation.

EP 0 169 601 A1

## COMPOSITION OCYTOCIQUE

La présente invention concerne une composition ocytocique destinée à accélérer les contractions utérines et activer le vêlage ou la mise bas des jeunes.

Elle trouve sa principale application en médecine vétérinaire pour accélérer le vêlage des vaches ou la mise bas des gorets chez les truies et pour traiter ou prévenir les infections mammaires, telles la fièvre du lait, les mammites des truies, l'hypogalactie chez les vaches laitières. Elle permet de guérir les infections de la matrice et des organes génitaux, suite à un vêlage ou mise bas difficile. Elle permet également d'entretenir la lactation et de favoriser la descente du lait immédiatement après la mise bas.

Les compositions ocytociques connues, obtenues par voie synthétique sont largement utilisées en médecine humaine, en particulier en obstétrique pour renforcer les contractions des muscles lisses, notamment de l'utérus, afin d'accélérer l'accouchement et pour arrêter les hémorragies du post-partum. Leur application s'est étendue également en médecine vétérinaire pour accélérer la mise bas et prévenir les infections de la matrice et les péritonites.

On sait également que, pour traiter lesdites infections chez les femelles, on ne peut pas faire appel à la pénicilline immédiatement après une mise bas. La pénicilline, qui possède une excellente efficacité à l'encontre desdites infections, présente en effet le grave inconvénient de réduire et même de supprimer la lactation.

Il faut donc faire appel à des antibiotiques plus évolués présentant un spectre plus large. On s'aperçoit cependant que nombre de produits ne sont pas efficaces contre lesdites infections.

On sait par un article de O.V. BUKHARIN et al intitulé "Effet antimicrobien d'une composition antibiotique contenant de l'ocytocine" que l'action de la streptomycine est renforcée en présence d'ocytocine.

Un effet synergique de l'ocytocine sur la streptomycine a été observé à l'encontre du staphylocoque sepsis lors du traitement d'infections suppurantes des tissus lisses. Ainsi, l'auteur affirme que les traitements chirurgicaux peuvent être remplacés par une paracentèse complétée par l'administration d'une dose d'antibiotique renforcée par de l'ocytocine.

Les quantités relatives d'antibiotiques et d'ocytocine mises en oeuvre par BUKHARIN semblent confirmer que celui-ci se limite à rechercher à renforcer l'action de l'antibiotique contre divers staphylocoques par l'adjonction de l'ocytocine.

L'article ne mentionne pas les problèmes de la lactation ni l'influence qu'exerce une composition antibiotique contenant de l'ocytocine.

Enfin, les doses d'antibiotiques sont nettement supérieures à celles d'ocytocine et semblent exagérées dans cet article.

La présente invention concerne une composition médicamenteuse contenant de l'ocytocine dans laquelle l'effet de l'ocytocine est renforcé par l'adjonction de faibles quantités d'antibiotiques.

La présente invention propose une composition antibiotique à usage vétérinaire utilement administrée lors de la mise bas pour prévenir les infections de la matrice et des glandes mammaires et prévenir la suppression de la lactation.

Elle est relative à une composition ocytocique destinée à accélérer les contractions utérines et à activer le vêlage ou la mise bas des jeunes bêtes, essentiellement caractérisée en ce qu'elle comporte dans des proportions convenables à la fois un dérivé de l'ocytocine et de la streptomycine.

Elle consiste à modifier les effets d'une composition ocytocique connue à l'aide d'un antibiotique.

Dans une forme de réalisation particulière, la composition ocytocique suivant l'invention contient du sulfate de streptomycine et de l'ocytocine dans un rapport pondéral compris entre 1,0 et 10.

D'autre particularités et détails de l'invention apparaîtront dans l'exemple suivant destiné à illustrer le caractère nouveau et inventif d'une composition suivant l'invention, sans que cependant ni

4    0169601

la composition précise ni la forme galénique ne doivent
être considérées comme une limitation.

On a préparé une forme galénique de la
composition antibiotique suivant l'invention en
mélangeant pour 100 ml les constituants suivants :

### TABLEAU I

Limites acceptables de composition d'une forme injectable d'un complexe ocytocique suivant l'invention.

- - - - - - - - - - - - - - - - - - - - - - - - -

| | |
|---|---|
| Sulfate de dihydro-streptomycine | 1.000 à 15.000 mg, étant entendu que 10.000 mg de sulfate de streptomycine correspondent à 7.500 mg de streptomycine de base |
| Borogluconate de calcium | 3.000 à 10.000 mg |
| Chlorure de magnésium | 1.000 à 2.500 mg |
| Chlorhydrate d'éphédrine | 3 à 8 mg |
| Thiomérosol | 3 à 8 mg |
| Ocytocine | 100 à 250 U.I. |
| Eau distillée pour injection parentérale | ad 100 ml. |

Une telle préparation peut être injectée à raison de 1 à 20 ml/100 kg de poids corporel, l'injection étant répétée une nouvelle fois plus tard dans un intervalle de 1 à 12 heures.

La teneur de certains constituants est de préférence celle indiquée par le tableau II.

**TABLEAU II**

Formule préférée de composition d'une forme injectable d'un complexe ocytocique suivant l'invention.

- - - - - - - - - - - - - - - - - - - - - - - - - - - - -

| Constituants | Par 100 ml |
|---|---|
| Sulfate de dihydro-streptomycine | 10.000 mg, étant entendu que 10.000 mg de ce composé correspondent à 7.500 mg de streptomycine de base |
| Borogluconate de calcium | 4.000 mg |
| Chlorure de magnésium | 1.200 mg |
| Chlorhydrate d'éphédrine | 4 mg |
| Thiomérosol | 4 mg |
| Ocytocine | 125 U.I. |
| Eau distillée pour injection parentérale | ad 100 ml |

Pour permettre une dissolution complète de tous les constituants du mélange, sans formation de dépôt, il est nécessaire de procéder de la manière suivante :

On dissout la quantité requise de streptomycine de base, sous forme de sulfate, soit 10 g dans 45 ml d'eau distillée, on y ajoute 12,5 ml de solution injectable d'ocytocine et, en dernier lieu, 40 ml d'une solution commerciale de borogluconate de calcium produit par AESCULAAP N.V./S.A, Gent - Belgique.

L'invention n'est donc pas limitée à la forme d'exécution stricte décrite ci-dessus. De nombreuses variantes et modifications peuvent être apportées à cette forme sans la soustraire du cadre de l'invention, déterminé par le fait que l'on y retrouve simultanément de la streptomycine et un composé ocytocique.

Ainsi, on pourrait utiliser d'autres excipients, diluants ou supports que ceux mentionnés dans les tableaux 1 et 2 et présenter la composition ocytocique susdite sous une forme galénique différente.

## REVENDICATIONS

1. Composition ocytocique destinée à accélérer les contractions utérines et à activer le vêlage ou la mise bas des jeunes, caractérisée en ce qu'elle comporte dans des proportions convenables à la fois du sulfate de streptomycine et de l'ocytocine.

2. Composition ocytocique suivant la revendication 1, caractérisée en ce qu'elle contient du sulfate de streptomycine et de l'ocytocine dans un rapport pondéral compris entre 1,0 et 10.

3. Composition ocytocique suivant l'une quelconque des revendications 1 ou 2, caractérisée en ce qu'elle contient, en tant que minéraux pour favoriser la production de lait, du borogluconate de calcium et du chlorure de magnésium.

4. Composition ocytocique suivant l'une quelconque des revendications précédentes, caractérisée en ce qu'elle comprend, pour 100 ml de solution à injecter, les constituants suivants :

| Constituants | Par 100 ml |
| --- | --- |
| Sulfate de dihydro-streptomycine | 1.000 à 15.000 mg étant entendu que 10.000 mg de sulfate de streptomycine correspondent à 7.500 mg de streptomycine de base. |
| Borogluconate de calcium | 3.000 à 10.000 mg |
| Chlorure de magnésium | 1.000 à 2.500 mg |

Chlorhydrate d'éphédrine          3 à   8 mg

Thiomérosol                       3 à   8 mg

Ocytocine                         100 à 250 U.I.

Eau distillée pour injection    ad 100 ml.
parentérale

5. Composition ocytocique suivant l'une quelconque des revendications précédentes, caractérisée en ce qu'elle comprend, pour 100 ml de solution à injecter, les constituants suivants :

| Constituants | Par 100 ml |
|---|---|
| Sulfate de dihydro-streptomycine | 10.000 mg, étant entendu que 10.000 mg de sulfate de strepto-mycine correspondent à 75 mg de strepcomycine de base |
| Borogluconate de calcium | 4.000 mg |
| Chlorure de magnésium | 1.200 mg |
| Chlorhydrate d'éphédrine | 4 mg |
| Thiomérosol | 4 mg |
| Ocytocine | 125 U.I. |
| Eau distillée pour injection parentérale | ad 100 ml. |

## Office européen des brevets

### RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| X | CHEMICAL ABSTRACTS, vol. 101, no. 3, 16 juillet 1984, page 29, no. 16896m, Columbus, Ohio, US; O.V. BUKHARIN et al.: "Potentiation of antimicrobial effect of antibiotics in combination with oxytocin" & ANTIBIOTIKI (MOSCOW) 1984, 29(5), 365-9 * Abrégé * | 1-2 | A 61 K 37/34<br>A 61 K 37/02 //<br>(A 61 K 37/34<br>A 61 K 31:71 )<br>(A 61 K 37/02<br>A 61 K 31:71 ) |
| A | UNLISTED DRUGS, vol. 28, no. 2, février 1976, page 28, no. d, Chatham, New Jersey, US; "P.O.P. Oxytocin" | 1-5 | |
| A | FR-A-2 358 160 (CESKOSLOVENSKA AKADEMIE VED) * Revendications * | 1-5 | |

**DOMAINES TECHNIQUES RECHERCHES (Int. Cl.4)**

A 61 K

Le présent rapport de recherche a été établi pour toutes les revendications

| Lieu de la recherche | Date d achèvement de la recherche | Examinateur |
|---|---|---|
| LA HAYE | 18-10-1985 | BRINKMANN C. |